# EUROPEAN PATENT SPECIFICATION

(11) **EP 4 408 498 B1**
(45) Date of publication and mention of the grant of the patent: **04.11.2026**
(21) Application number: 22792890.0
(22) Date of filing: 26.09.2022
(51) Int. Cl.: A61M 1/36, A61M 1/00, A61M 3/02, A61M 25/00

(54) **CANNULA FOR THE DRAINAGE OF BLOOD VESSELS**
KANÜLE ZUR DRAINAGE VON BLUTGEFÄSSEN
CANULE POUR LE DRAINAGE DE VAISSEAUX SANGUINS

(30) Priority: 27.09.2021 IT 202100024617
(43) Date of publication of application: 07.08.2024
(73) Proprietor: Eurosets S.r.l., 41036 Medolla (MO) (IT)
(72) Inventor: GHELLI, Nicola, 41036 Medolla (MO) (IT); PICCININI, Marco, 41036 Medolla (MO) (IT)
(74) Representative: Zoli, Filippo
(86) International application number: PCT/IB2022/059114
(87) International publication number: WO 2023/047372

(56) References cited:
- EP-A1- 3 656 414
- US-A- 4 863 441
- US-A1- 2011 130 745
- US-A1- 2012 078 226
- US-A1- 2014 094 741

## Description

### Technical Field

The present invention relates to a cannula for the drainage of blood vessels.

### Background Art

To date, the use of cannulas for the drainage of blood vessels during extracorporeal circulation (ECC) is widely used.

Cannulas can be of the venous or arterial type depending on whether they are used to drain venous or arterial blood, so as to convey it from the patient to the extra-corporeal circuit and from the extra-corporeal circuit to the patient, respectively.

Cannulas can also be used during various types of operations, such as e.g. laparoscopic, endoscopic and arthroscopic operations, to suck in fluids or to carry out perfusions.

Cannulas of known type comprise at least one tubular body provided with a stretch of extremity intended to be inserted into a blood vessel and on which a plurality of apertures are defined which are adapted to allow the passage of fluid substances.

**In** more detail, the tubular body defines an internal channel into which the fluid substances flow.

It is well known that cannulas must ensure optimal fluid suction and drainage of adequate volumes of blood so that the extracorporeal circulation equipment can effectively provide for the treatment of the blood itself, i.e., the removal of carbon dioxide and subsequent oxygenation.

Insufficient or discontinuous suction can cause serious damage to the patient's tissues and organs.

For this purpose, the apertures are appropriately shaped and arranged along the tubular body in order to increase the fluid suction capacity of the cannula.

Known cannulas, however, do have some drawbacks mainly related to the fact that if the aperture is substantially in contact with the blood vessel wall, the flow rate of fluid through it is significantly reduced with respect to the nominal flow rate, so it needs to be repositioned to achieve optimal operating conditions.

This process involves a longer timeline and, in addition, can cause damage to the blood vessel itself.

In addition, the presence of a large number of apertures may result in weakening of the cannula structure and lower resistance to mechanical stresses. Cannulas for the drainage of blood vessels comprising apertures for the passage of fluid having a variable cross-section are known from US 4863441 A1, US 2014/094741 A1, US 2012/078226 A1 and EP 3656414 A1.

### Description of the Invention

The main aim of the present invention is to devise a cannula for the drainage of blood vessels that allows effective suction of fluid substances.

Another object of the present invention is to devise a cannula for the drainage of blood vessels that allows maintaining a constant and adequate suction flow rate throughout the entire treatment.

A further object of the present invention is to devise a cannula for the drainage of blood vessels that is provided with high resistance to mechanical stress.

Another object of the present invention is to devise a cannula for the drainage of blood vessels that can overcome the aforementioned drawbacks of the prior art within the framework of a simple and rational solution that is also easy and effective to use as well as inexpensive.

The aforementioned objects are achieved by this cannula for the drainage of blood vessels having the characteristics of claim 1.

### Brief Description of the Drawings

Other characteristics and advantages of the present invention will become more apparent from the description of a preferred, but not exclusive, embodiment of a cannula for the drainage of blood vessels, illustrated by way of an indicative, yet non-limiting example, in the attached tables of drawings in which:
Figure 1 is an axonometric view of a cannula in accordance with a first embodiment;
Figures 2 and 3 are side views of the cannula in Figure 1 from two different angles;
Figure 4 is an axonometric view of a cannula in accordance with a second embodiment;
Figures 5 and 6 are side views of the cannula in Figure 4 from two different angles;
Figure 7 is an axonometric view of a cannula according to the invention, in accordance with a third embodiment;
Figure 8 is a front view of the cannula in Figure 7;
Figures 9 and 10 are side views of the cannula in Figure 7 from two different angles;
Figure **11** is an axonometric view of a cannula according to the invention, in accordance with a fourth embodiment;
Figure 12 is a front view of the cannula in Figure 11;
Figure 13 is an axonometric view of a cannula according to the invention, in accordance with a fifth embodiment;
Figure 14 is a front view of the cannula in Figure 13;
Figure 15 is an axonometric view of a cannula according to the invention, in accordance with a sixth embodiment;
Figure 16 is a front view of the cannula in Figure 15.

### Embodiments of the Invention

With particular reference to these figures, reference numeral 1 globally indicates a cannula for the drainage of blood vessels.

The cannula 1 comprises at least one tubular body 2 provided with a stretch of extremity 3 intended to be fitted into a blood vessel and on which a plurality of apertures for the passage of fluid substances is defined.

In the context of this disclosure, the expression "fluid substances" refers to arterial or venous blood and/or to other body fluids that can be sucked in, for example, during laparoscopic, endoscopic or arthroscopic treatments. It also refers to fluids that can be administered by perfusion, e.g., medicaments.

The tubular body 2 is of substantially cylindrical shape and comprises a cylindrical side wall defining an internal channel for the passage of the fluid substances.

The apertures are in fluidic communication with the passage channel.

According to the invention, the apertures comprise at least one pair of first apertures 4 having a variable cross-section, the cross-sections of which are converging along relevant directions of development opposite each other.

The first apertures 4 are defined on the side wall of the tubular body 2.

In more detail, each first aperture 4 has a cross section the width of which is decreasing along the relevant direction of development.

Thus, the first apertures 4 have a convergent conformation along the relevant direction of development.

Usefully, the directions of development of the first apertures 4 are substantially parallel to the longitudinal axis A of the tubular body 2.

The conformation of the first apertures 4 allows the fluid substances to be conveyed in an optimal manner within the passage channel defined by the tubular body 2.

In the preferred embodiments shown in the figures, each of the first apertures 4 is of triangular conformation.

In addition, each of the first apertures 4 is of symmetrical conformation with respect to a longitudinal median plane P1,P2 of the tubular body 2 passing through the relevant direction of development. In other words, the first apertures 4 are conformed substantially as an isosceles triangle.

Alternative embodiments cannot however be ruled out in which the first apertures 4 have a different conformation, e.g., trapezoidal or teardrop-shaped. The first apertures 4 have the same conformation and the same passage area for the fluid substances.

It cannot, however, be ruled out that the first apertures have different conformations to each other and/or different passage areas for the fluid substances.

Advantageously, the portion of the stretch of extremity 3 on which the first apertures 4 are defined is convex.

In more detail, each first aperture 4 defines a recess in the side wall of the stretch of extremity 3. In even more detail, the recess is deeper where there is the portion of the first aperture 4 with the largest cross section than where there is the portion of the first aperture 4 with the smallest cross section.

Usefully, each first aperture 4 defines a base edge 5 substantially parallel to a reference plane T substantially perpendicular to the longitudinal axis A.

The first aperture 4 also has a pair of side edges 6, contiguous with the base edge 5 and converging with each other towards the vertex of the triangle.

The side edges 6 converging with each other of each of the first apertures 4 extend along the aforementioned convex portion.

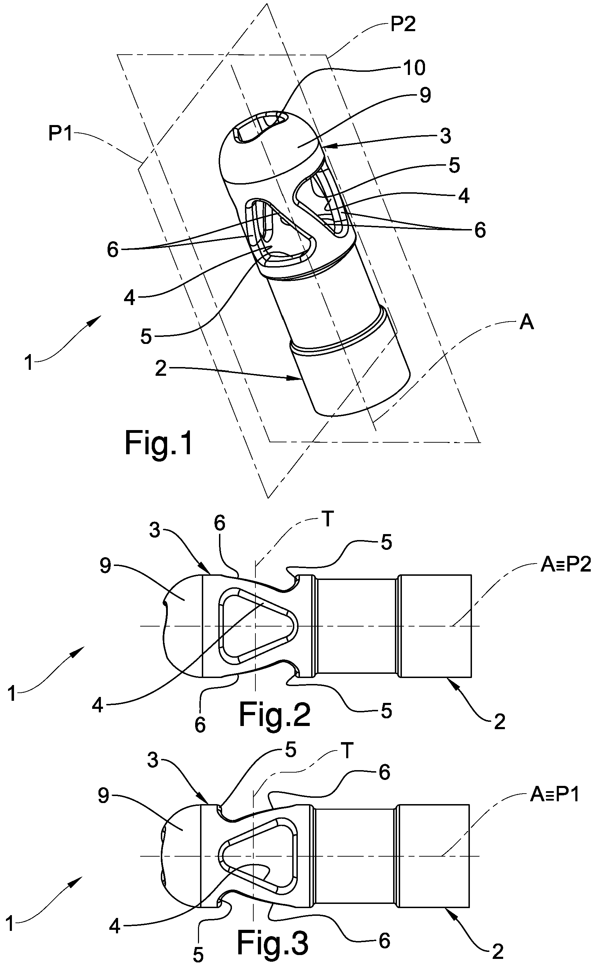

In accordance with the embodiments shown in Figures 1 through 6, the first apertures 4 of each pair of first apertures are arranged angularly offset from each other along the perimeter development of the tubular body 2. More particularly, in these embodiments, the cannula 1 comprises a plurality of pairs of first apertures 4, two pairs specifically, where each first aperture 4 has a direction of development oriented in the opposite way to the first aperture 4 consecutive thereto along the perimeter development of the tubular body 2.

Specifically, the first apertures 4 are offset from each other by an angle substantially equal to 90°.

With particular reference to the first and to the second embodiments shown in Figures 1 through 6, two first apertures 4 are symmetrical with respect to a first longitudinal median plane P1 and the other two first apertures 4 are symmetrical with respect to a second longitudinal median plane P2 perpendicular to the first plane P1.

The directions of development of the first apertures 4 are substantially parallel and separate from each other.

In particular, Figures 1 to 3 show a first embodiment wherein the first apertures 4 are arranged side by side along the perimeter development of the tubular body 2.

In more detail, the tubular body 2 has a substantially circular cross section and the first apertures 4 are arranged along the same circumference.

As stated above, the directions of development of the first apertures 4 are oriented in opposite way to each other. The first apertures 4, therefore, are arranged so that the portion with the largest cross section of one of the first apertures 4 is side by side to the portion having the smallest cross section of the first apertures 4 adjacent thereto.

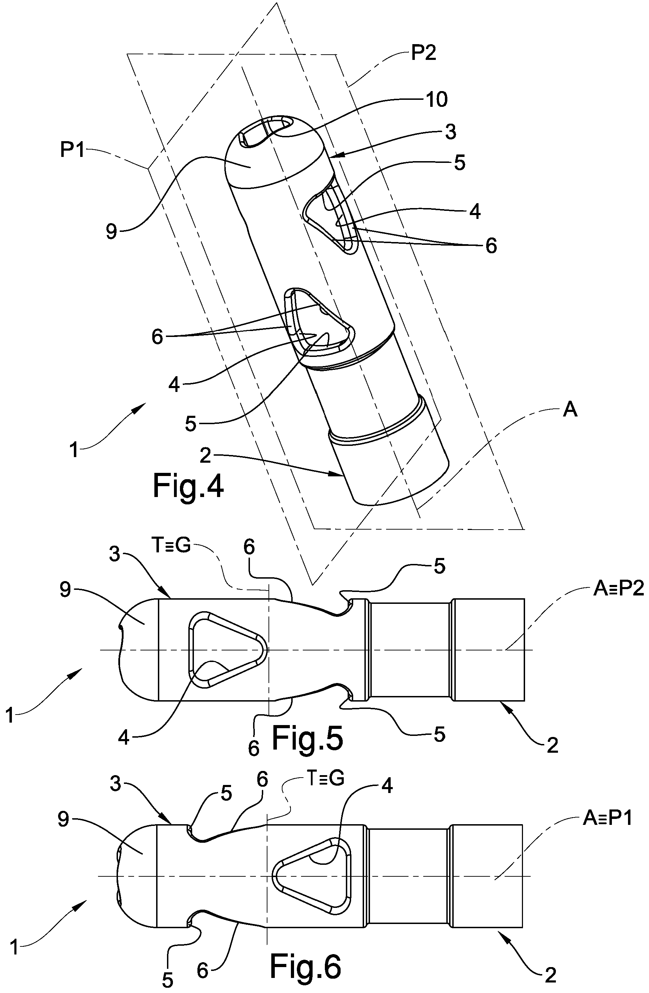

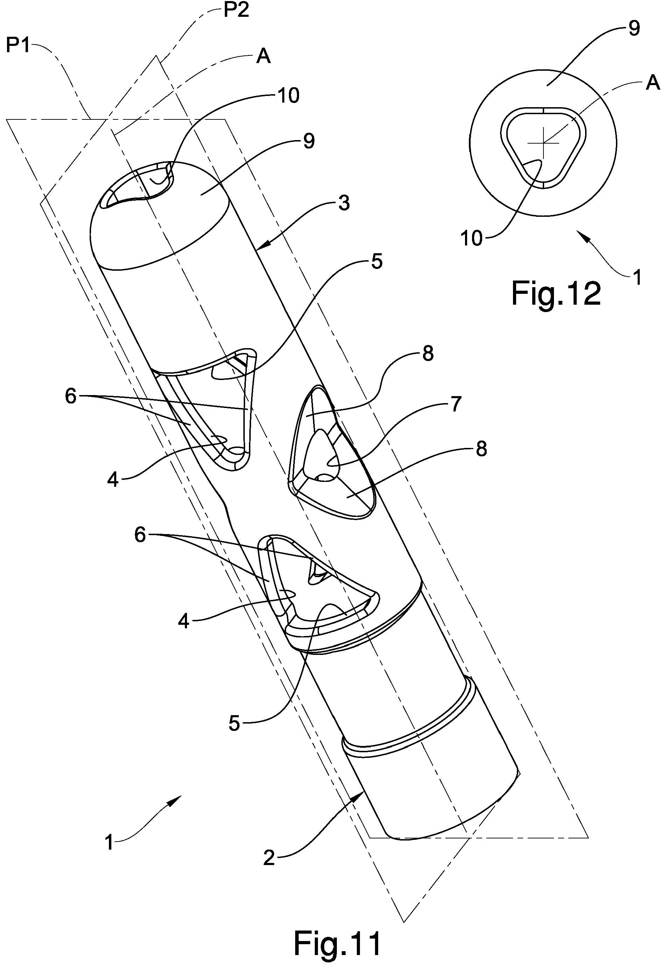

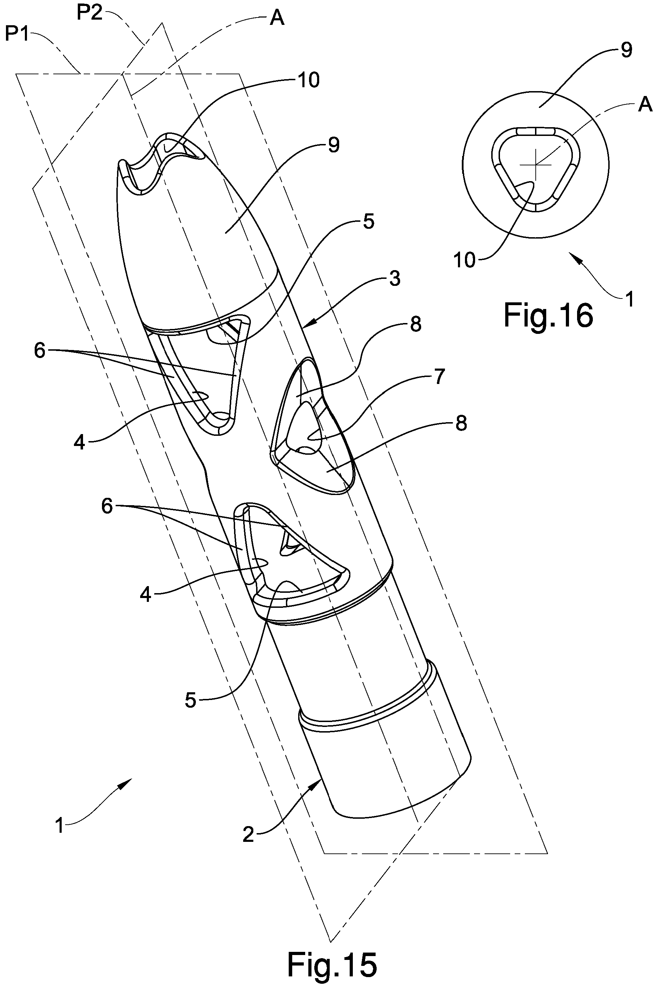

In the embodiments shown in Figures 4 to 16, the first apertures 4 of each pair are arranged from opposite sides with respect to a transverse plane G located between them, substantially perpendicular to said longitudinal axis A and parallel to the relevant base edges 5. **In** these embodiments, the transverse plane G thus coincides with the aforementioned reference plane T.

More particularly, in Figures 4 through 6, on the other hand, a second embodiment is shown in which the first apertures 4 are further arranged staggered longitudinally with each other.

**In** other words, the first apertures 4 of each pair lie on two separate circumferences.

**In** addition, the first apertures 4 of each pair are arranged converging towards each other. Moreover, the first apertures 4 are arranged converging towards the transverse plane G. **In** other words, the direction of development of each first aperture 4 is oriented towards the first apertures 4 consecutive thereto along the perimeter development of the tubular body 2.

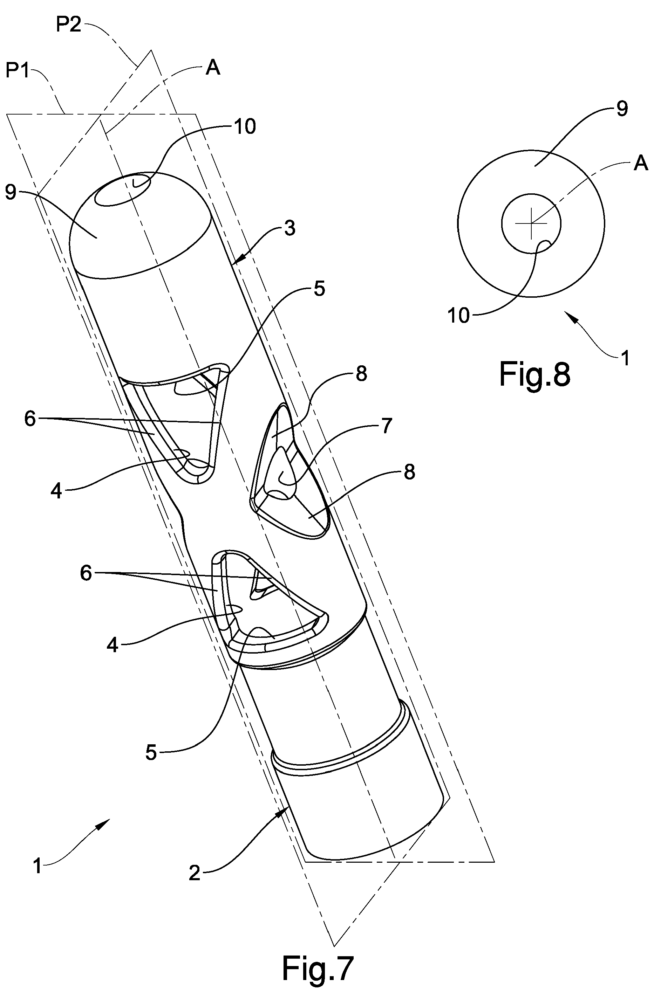

**In** the further embodiments, shown in Figures 7 to 16, the first apertures 4 of each pair of first apertures are arranged in succession to each other parallel to the longitudinal axis A.

**In** these embodiments, the directions of development of the first apertures 4 are, therefore, aligned with each other.

**In** particular, the first apertures 4 are arranged converging towards each other.

**In** other words, the smallest portions of the first apertures 4 are placed at a shorter distance from each other than the largest portions of the first apertures 4. The first apertures 4 are arranged facing each other at a relevant vertex.

Specifically, the distance between the relevant vertices is less than the distance between the bases opposite these vertices.

The first apertures 4 of each pair are arranged symmetrically to each other with respect to the transverse plane G.

Advantageously, the tubular body 2 comprises at least two pairs of first apertures 4.

With particular reference to the embodiments shown in Figures 7 to 16, the tubular body 2 comprises at least two pairs of first apertures 4 arranged symmetrically to each other with respect to the first longitudinal median plane P1 of the tubular body itself. The first apertures 4 give the portion of the stretch of extremity 3 a convex conformation, not protruding from the side wall of the tubular body 2 which allows the optimal passage of the fluid substances even when the apertures themselves are placed close to the blood vessel's wall.

Again with reference to the third embodiment, the tubular body 2 also advantageously comprises at least a second aperture 7 of ovoidal conformation, staggered longitudinally with respect to the first apertures 4.

The second aperture 7 is, therefore, misaligned with respect to each pair of first apertures 4 aligned with each other.

In more detail, the second aperture 7 is angularly staggered with respect to the first apertures 4 along the perimeter development of the stretch of extremity 3.

Preferably, the second aperture 7 is staggered by an angle substantially equal to 90° with respect to the first apertures 4.

The second aperture 7 is also defined at a portion of the stretch of extremity 3 positioned between the first apertures 4.

Usefully, the tubular body 2 comprises a pair of flat cutting surfaces 8, formed on the stretch of extremity 3, which are inclined with respect to the longitudinal axis A and converging with each other, defining the second aperture 7.

In more detail, the cutting surfaces 8 are incident with each other at the transverse plane G.

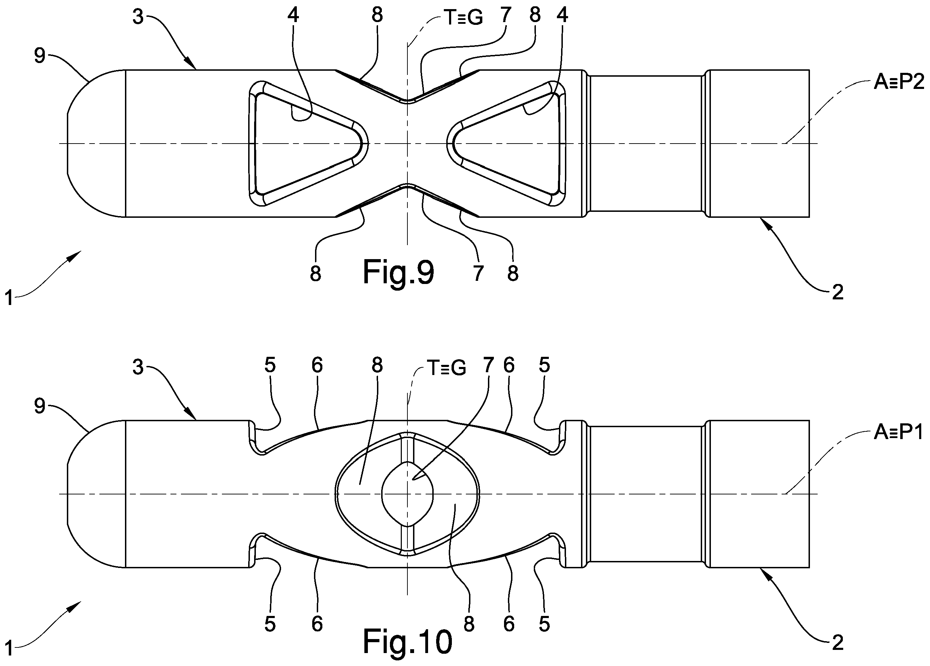

As also shown in Figure 9, the second aperture 7 is, therefore, equidistant from the first apertures 4 of each pair.

The cutting surfaces 8 are arranged substantially perpendicular to the first plane P1. In fact, as shown above, the second aperture 7 is angularly staggered by an angle substantially equal to 90° with respect to the first apertures 4.

The cutting surfaces 8 define an angle between them with amplitude of between 120° to 150°.

Such an arrangement of the cutting surfaces 8 tends to facilitate the flow of the fluid substances towards the second aperture 7.

Advantageously, the tubular body 2 comprises at least two second apertures 7 arranged symmetrically to each other with respect to a second longitudinal median plane P2 substantially perpendicular to the first plane P1.

The two second apertures 7 are defined, therefore, on opposite sides of the stretch of extremity 3.

The second apertures 7 help to give the stretch of extremity 3 an "X" conformation that allows ensuring an optimal flow rate of the fluid substances even in the event of the apertures being positioned close to the blood vessel's wall.

Usefully, the stretch of extremity 3 comprises a distal portion 9 provided with at least one extremal hole 10.

The distal portion 9 is adapted to facilitate the insertion of the stretch of extremity 3 inside the blood vessel and the extremal hole 10 helps, in turn, to ensure an adequate passage of the fluid substances.

The distal portion 9 has a conformation selected between: hemispherical and ogival.

In accordance with the embodiments shown in Figures 1 to 12, the distal portion 9 has a substantially hemispherical conformation.

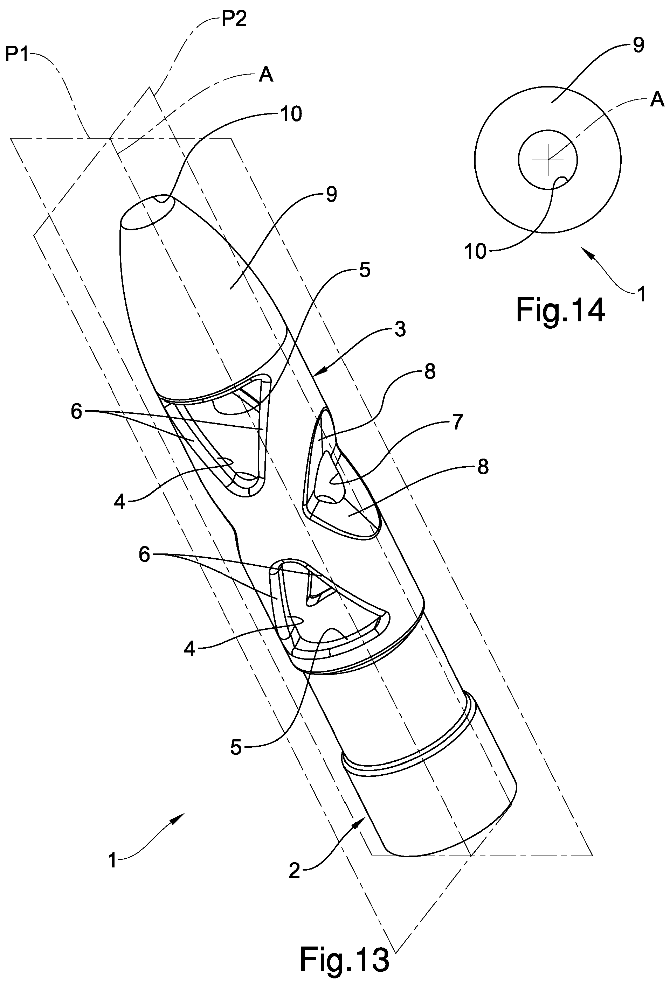

**In** accordance with the alternative embodiments shown in Figures 13 to 16, on the other hand, the distal portion 9 has a substantially ogival conformation.

The extremal hole 10 has a cross-section with a conformation selected between circular and triangular.

**In** accordance with the embodiments shown in Figures 7-10 and 13-14, the extremal hole 10 has a substantially circular cross-section.

**In** accordance with the alternative embodiments shown in Figures 1-6 and 11-12 and 15-16, on the other hand, the extremal hole 10 has a substantially triangular cross-section.

It has in practice been ascertained that the described invention achieves the intended objects, and in particular, the fact is emphasized that the present cannula for the drainage of blood vessels allows an effective passage of fluid substances and allows a constant and adequate suction flow rate to be maintained throughout the treatment.

The special combination of the first apertures and of the second apertures gives the cannula a conformation that allows blood to be conveyed in an optimal manner within the passage channel and, at the same time, high resistance to mechanical stresses.

## Claims

1. Cannula (1) for the drainage of blood vessels, comprising at least one tubular body (2) provided with a stretch of extremity (3) intended to be fitted into a blood vessel and on which a plurality of apertures for the passage of fluid substances is defined, wherein said apertures comprise at least one pair of first apertures (4) having a variable cross-section, the cross-sections of which are converging along relevant directions of development opposite each other, said directions of development being substantially parallel to the longitudinal axis (A) of said tubular body (2),
wherein said first apertures (4) are each of substantially triangular conformation, where each first aperture (4) defines a base edge (5) substantially parallel to a reference plane (T) substantially perpendicular to the longitudinal axis (A), said first aperture (4) also having a pair of side edges (6), contiguous with the base edge (5) and converging with each other towards the vertex of the triangle,
**characterized by** the fact that the first apertures (4) of said pair are arranged in succession to each other parallel to said longitudinal axis (A), said directions of development being aligned with each other, and
by the fact that the first apertures (4) of said pair are arranged symmetrically to each other with respect to a transverse plane (G) substantially perpendicular to said longitudinal axis (A).

2. Cannula (1) according to claim 1, **characterized by** the fact that the portion of said stretch of extremity (3) on which said first apertures (4) are defined is convex.

3. Cannula according to claim 2, **characterized by** the fact that said side edges (6) extend on said convex portion.

4. Cannula (1) according to one or more of the preceding claims, **characterized by** the fact that the first apertures (4) of said pair are arranged with their relevant sections converging towards each other.

5. Cannula (1) according to one or more of the preceding claims, **characterized by** the fact that said tubular body (2) comprises at least two of said pairs of first apertures (4) arranged symmetrically to each other with respect to a first longitudinal median plane (P1) of said tubular body (2).

6. Cannula (1) according to one or more of the preceding claims, **characterized by** the fact that said tubular body (2) comprises at least one second aperture (7) of ovoidal conformation, staggered with respect to said first apertures (4) along the longitudinal development of said tubular body (2).

7. Cannula (1) according to claim 6, **characterized by** the fact that said second aperture (7) is angularly staggered with respect to said first apertures (4) along the perimeter development of said tubular body (2).

8. Cannula (1) according to claim 6 or 7, **characterized by** the fact that said second aperture (7) is defined at a portion of said stretch of extremity (3) positioned between said first apertures (4).

9. Cannula (1) according to one or more of claims 6 to 8, **characterized by** the fact that said tubular body (2) comprises a pair of flat cutting surfaces (8), formed on said stretch of extremity (3), inclined with respect to said longitudinal axis (A) and converging with each other, thus defining said second aperture (7).

10. Cannula (1) according to claim 9, **characterized by** the fact that said cutting surfaces (8) are incident with each other at said transverse plane (G).

11. Cannula (1) according to claim 5 and claim 9 or 10, **characterized by** the fact that said cutting surfaces (8) are arranged substantially perpendicular to said first plane (P1).

12. Cannula (1) according to claim 5 and one or more of claims 6 to 11, **characterized by** the fact that said tubular body (2) comprises at least two of said second apertures (7) arranged symmetrically to each other with respect to a second longitudinal median plane (P2) substantially perpendicular to said first plane (P1).

13. Cannula (1) according to one or more of the preceding claims, **characterized by** the fact that said stretch of extremity (3) comprises a distal portion (9) provided with at least one extremal hole (10), wherein said distal portion (9) has a conformation selected between hemispherical and ogival and wherein said extremal hole (10) has a conformation cross-section selected between circular or triangular.

## Patentansprüche

1. Kanüle (1) zur Drainage von Blutgefäßen, umfassend wenigstens einen rohrförmigen Körper (2), der mit einem Endabschnitt (3) versehen ist, der dazu bestimmt ist, in ein Blutgefäß eingesetzt zu werden, und an dem eine Mehrzahl von Öffnungen zum Durchtritt fluider Substanzen ausgebildet ist, wobei die Öffnungen wenigstens ein Paar erster Öffnungen (4) mit veränderlichem Querschnitt umfassen, deren Querschnitte entlang jeweiliger, einander entgegengesetzter Erstreckungsrichtungen konvergieren, wobei die Erstreckungsrichtungen im Wesentlichen parallel zu der Längsachse (A) des rohrförmigen Körpers (2) sind,
wobei die ersten Öffnungen (4) jeweils eine im Wesentlichen dreieckige Form aufweisen, wobei jede erste Öffnung (4) einen Basisrand (5) definiert, der im Wesentlichen parallel zu einer Bezugsebene (T) ist, die im Wesentlichen senkrecht zu der Längsachse (A) ist, wobei die erste Öffnung (4) ferner ein Paar Seitenränder (6) aufweist, die an den Basisrand (5) angrenzen und zueinander in Richtung des Scheitelpunkts des Dreiecks konvergieren,
**dadurch gekennzeichnet, dass** die ersten Öffnungen (4) des Paars aufeinanderfolgend zueinander parallel zu der Längsachse (A) angeordnet sind, wobei die Erstreckungsrichtungen miteinander fluchten, und
dadurch, dass die ersten Öffnungen (4) des Paars symmetrisch zueinander bezüglich einer Querebene (G) angeordnet sind, die im Wesentlichen senkrecht zu der Längsachse (A) ist.

2. Kanüle (1) nach Anspruch 1, **dadurch gekennzeichnet, dass** der Abschnitt des Endabschnitts (3), an dem die ersten Öffnungen (4) ausgebildet sind, konvex ist.

3. Kanüle nach Anspruch 2, **dadurch gekennzeichnet, dass** sich die Seitenränder (6) auf dem konvexen Abschnitt erstrecken.

4. Kanüle (1) nach einem oder mehreren der vorhergehenden Ansprüche, **dadurch gekennzeichnet, dass** die ersten Öffnungen (4) des Paars mit ihren jeweiligen Abschnitten aufeinander zu konvergierend angeordnet sind.

5. Kanüle (1) nach einem oder mehreren der vorhergehenden Ansprüche, **dadurch gekennzeichnet, dass** der rohrförmige Körper (2) wenigstens zwei der Paare erster Öffnungen (4) umfasst, die symmetrisch zueinander bezüglich einer ersten Längsmittelebene (P1) des rohrförmigen Körpers (2) angeordnet sind.

6. Kanüle (1) nach einem oder mehreren der vorhergehenden Ansprüche, **dadurch gekennzeichnet, dass** der rohrförmige Körper (2) wenigstens eine zweite Öffnung (7) mit ovoidaler Form umfasst, die bezüglich der ersten Öffnungen (4) entlang der Längserstreckung des rohrförmigen Körpers (2) versetzt ist.

7. Kanüle (1) nach Anspruch 6, **dadurch gekennzeichnet, dass** die zweite Öffnung (7) bezüglich der ersten Öffnungen (4) entlang der Umfangserstreckung des rohrförmigen Körpers (2) winkelversetzt ist.

8. Kanüle (1) nach Anspruch 6 oder 7, **dadurch gekennzeichnet, dass** die zweite Öffnung (7) an einem Abschnitt des Endabschnitts (3) ausgebildet ist, der zwischen den ersten Öffnungen (4) positioniert ist.

9. Kanüle (1) nach einem oder mehreren der Ansprüche 6 bis 8, **dadurch gekennzeichnet, dass** der rohrförmige Körper (2) ein Paar ebener Schnittflächen (8) umfasst, die an dem Endabschnitt (3) ausgebildet, bezüglich der Längsachse (A) geneigt und zueinander konvergierend sind und dadurch die zweite Öffnung (7) definieren.

10. Kanüle (1) nach Anspruch 9, **dadurch gekennzeichnet, dass** die Schnittflächen (8) in der Querebene (G) aufeinandertreffen.

11. Kanüle (1) nach Anspruch 5 und Anspruch 9 oder 10, **dadurch gekennzeichnet, dass** die Schnittflächen (8) im Wesentlichen senkrecht zu der ersten Ebene (P1) angeordnet sind.

12. Kanüle (1) nach Anspruch 5 und einem oder mehreren der Ansprüche 6 bis 11, **dadurch gekennzeichnet, dass** der rohrförmige Körper (2) wenigstens zwei der zweiten Öffnungen (7) umfasst, die symmetrisch zueinander bezüglich einer zweiten Längsmittelebene (P2) angeordnet sind, die im Wesentlichen senkrecht zu der ersten Ebene (P1) ist.

13. Kanüle (1) nach einem oder mehreren der vorhergehenden Ansprüche, **dadurch gekennzeichnet, dass** der Endabschnitt (3) einen distalen Abschnitt (9) umfasst, der mit wenigstens einem endseitigen Loch (10) versehen ist, wobei der distale Abschnitt (9) eine Form aufweist, die zwischen halbkugelförmig und ogival ausgewählt ist, und wobei das endseitige Loch (10) eine Querschnittsform aufweist, die zwischen kreisförmig oder dreieckig ausgewählt ist.

## Revendications

1. - Canule (1) pour le drainage de vaisseaux sanguins, comprenant au moins un corps tubulaire (2) comportant une extension d'extrémité (3) destinée à être introduite dans un vaisseau sanguin et sur laquelle une pluralité d'ouvertures pour le passage de substances fluides est définie, lesdites ouvertures comprenant au moins une paire de premières ouvertures (4) ayant une section transversale variable, dont les sections transversales convergent le long de directions de développement correspondantes opposées l'une à l'autre, lesdites directions de développement étant sensiblement parallèles à l'axe longitudinal (A) dudit corps tubulaire (2),
dans laquelle lesdites premières ouvertures (4) présentent chacune une conformation sensiblement triangulaire, où chaque première ouverture (4) définit un bord de base (5) sensiblement parallèle à un plan de référence (T) sensiblement perpendiculaire à l'axe longitudinal (A), ladite première ouverture (4) ayant également une paire de bords latéraux (6), contigus au bord de base (5) et convergeant l'un vers l'autre vers le sommet du triangle,
**caractérisée par le fait que** les premières ouvertures (4) de ladite paire sont disposées en se succédant l'une à l'autre parallèlement audit axe longitudinal (A), lesdites directions de développement étant alignées l'une par rapport à l'autre, et
**par le fait que** les premières ouvertures (4) de ladite paire sont disposées symétriquement l'une à l'autre par rapport à un plan transversal (G) sensiblement perpendiculaire audit axe longitudinal (A).

2. - Canule (1) selon la revendication 1, **caractérisée par le fait que** la partie de ladite extension d'extrémité (3) sur laquelle lesdites premières ouvertures (4) sont définies est convexe.

3. - Canule selon la revendication 2, **caractérisée par le fait que** lesdits bords latéraux (6) s'étendent sur ladite partie convexe.

4. - Canule (1) selon l'une ou plusieurs des revendications précédentes, **caractérisée par le fait que** les premières ouvertures (4) de ladite paire sont disposées avec leurs sections correspondantes convergeant l'une vers l'autre.

5. - Canule (1) selon l'une ou plusieurs des revendications précédentes, **caractérisée par le fait que** ledit corps tubulaire (2) comprend au moins deux desdites paires de premières ouvertures (4) disposées symétriquement l'une à l'autre par rapport à un premier plan médian longitudinal (P1) dudit corps tubulaire (2).

6. - Canule (1) selon l'une ou plusieurs des revendications précédentes, **caractérisée par le fait que** ledit corps tubulaire (2) comprend au moins une seconde ouverture (7) de conformation ovoïde, décalée par rapport auxdites premières ouvertures (4) le long du développement longitudinal dudit corps tubulaire (2).

7. - Canule (1) selon la revendication 6, **caractérisée par le fait que** ladite seconde ouverture (7) est décalée angulairement par rapport auxdites premières ouvertures (4) le long du développement périphérique dudit corps tubulaire (2).

8. - Canule (1) selon la revendication 6 ou 7, **caractérisée par le fait que** ladite seconde ouverture (7) est définie à une partie de ladite extension d'extrémité (3) positionnée entre lesdites premières ouvertures (4).

9. - Canule (1) selon l'une ou plusieurs des revendications 6 à 8, **caractérisée par le fait que** ledit corps tubulaire (2) comprend une paire de surfaces de coupe planes (8), formées sur ladite extension d'extrémité (3), inclinées par rapport audit axe longitudinal (A) et convergeant l'une vers l'autre, définissant ainsi ladite seconde ouverture (7).

10. - Canule (1) selon la revendication 9, **caractérisée par le fait que** lesdites surfaces de coupe (8) sont incidentes l'une à l'autre audit plan transversal (G).

11. - Canule (1) selon la revendication 5 et la revendication 9 ou 10, **caractérisée par le fait que** lesdites surfaces de coupe (8) sont disposées sensiblement perpendiculairement audit premier plan (P1).

12. - Canule (1) selon la revendication 5 et l'une ou plusieurs des revendications 6 à 11, **caractérisée par le fait que** ledit corps tubulaire (2) comprend au moins deux desdites secondes ouvertures (7) disposées symétriquement l'une à l'autre par rapport à un second plan médian longitudinal (P2) sensiblement perpendiculaire audit premier plan (P1).

13. - Canule (1) selon l'une ou plusieurs des revendications précédentes, **caractérisée par le fait que** ladite extension d'extrémité (3) comprend une partie distale (9) comportant au moins un trou extrême (10), ladite portion distale (9) présentant une conformation choisie entre hémisphérique et ogivale et ledit trou extrême (10) présentant une section transversale de conformation choisie entre circulaire et triangulaire.
